# FASCICULE DE BREVET EUROPEEN

(11) **EP 1 139 928 B1**
(45) Date de publication et mention de la délivrance du brevet: **13.09.2006**
(21) Numéro de dépôt: 99958312.3
(22) Date de dépôt: 13.12.1999
(51) Int. Cl.: A61F 2/42, A61B 17/86

(54) **PROTHESE TOTALE METATARSO-PHALANGIENNE A COUPE**
METATARSALPHALANGEALE TOTALPROTHESE FÜR VORGESCHNITTENE KNOCHENOBERFLÄCHEN
TOTAL METATARSOPHALANGEAL PROSTHESIS WITH SECTION PLANE

(30) Priorité: 14.12.1998 FR 9815774
(43) Date de publication de la demande: 10.10.2001
(73) Titulaire: Depuy (Ireland) Limited, Ringaskiddy, County Cork (IE)
(72) Inventeur: BAROUK, Louis, Samuel, F-33370 Yvrac (FR); COULANGE, Vincent, F-69100 Villeurbanne (FR); GAUME, Jean-Michel, F-42300 Roanne (FR); AUGOYARD, Marc, F-69160 Tassin la Demi Lune (FR); PEYROT, Jacques, F-69160 Tassin la Demi Lune (FR); BENICHOU, Michel, F-34090 Montpellier (FR); MAESTRO, Michel, Dom. de l'Orangeraie, Villa l'Olivier, 06200 Nice (FR); RAGUSA, Mathieu, F-38240 Meylan (FR); VALTIN, Bernard, F-94500 Champigny (FR)
(74) Mandataire: Bernasconi, Jean Raymond
(86) Numéro de dépôt international: PCT/FR1999/003113
(87) Numéro de publication internationale: WO 2000/035381

(56) Documents cités:
- EP-A- 0 572 339
- WO-A-92/00709
- WO-A-96/37169
- DE-A- 3 017 798
- FR-A- 2 697 155
- FR-A- 2 709 948
- FR-A- 2 733 412
- FR-A- 2 734 150
- US-A- 5 037 440
- US-A- 5 458 648

## Description

La présente invention a pour objet une prothèse totale métatarso-phalangienne à coupe, du type comprenant une prothèse métatarsienne à tige d'ancrage osseux, une prothèse phalangienne et un plateau articulaire adapté pour être disposé sur la prothèse phalangienne, ce plateau étant pourvu d'une surface articulaire coopérant avec une surface complémentaire d'articulation de la prothèse métatarsienne.

Ce type de prothèse est adapté pour être disposé dans le gros orteil entre la phalange et le métatarsien, afin de remédier à certaines pathologies, à savoir l'hallux rigidus voire l'hallux valgus arthrosique.

On sait que l'hallux valgus consiste en une déformation latérale du gros orteil, associée à une déviation médiale du premier métatarsien ou métatarsus varus, et qui provoquent la formation de bursites latérales douloureuses. La seconde pathologie concernée, l'hallux rigidus, est associé à une arthrose et révélée par un pincement de l'interligne (espace entre le métatarsien et la phalange,), le cartilage ayant partiellement ou totalement disparu sur les faces en regard de l'articulation. Les chirurgiens distinguent trois stades de l'hallux rigidus : le stade I très peu évolué, peut être traité par une ostéotomie du métatarsien qui permet d'exercer des effets de raccourcissement, d'abaissement. On peut y associer également un accourcissement de la première phalange du gros orteil toujours dans le but d'augmenter l'effet de détente longitudinale.

Le stade II correspond à une usure plus prononcée des cartilages, mais l'articulation présente encore une petite mobilité. Le traitement chirurgical peut être soit celui du stade précédent, soit la pose d'une prothèse. Enfin au stade III il ne reste plus aucun cartilage pour assurer l'articulation, la mobilité est quasi nulle et de vives douleurs apparaissent. Le seul traitement chirurgical possible est alors l'arthrodèse ou la prothèse.

Il existe des prothèses totales métatarso-phalangiennes « à coupe » et « de resurfaçage ». Les prothèses « à coupe » impliquent la coupe préliminaire sur le métatarsien et la phalange de surfaces planes destinées à recevoir les composants de la prothèse. Par contre les prothèses « de resurfaçage » ne nécessitent qu'un resurfaçage des surfaces articulaires en regard du métatarsien et de la phalange car elles sont profilées pour ne remplacer que le cartilage.

On connaît des prothèses totales pour gros orteils, par exemple par le brevet US-A-5 458 648 et les brevets FR-A-2 709 948 et 2 697 155. Ces prothèses ne donnent pas entièrement satisfaction notamment du fait qu'elles ne s'adaptent à l'anatomie naturelle du patient que de manière imparfaite.

L'invention a pour but de proposer une prothèse totale métatarso-phalangienne agencée pour s'adapter au mieux à l'anatomie de l'articulation métatarso-phalangienne, et qui en outre présente les degrés de liberté voulus pour restituer au plus près l'articulation naturelle dans les différentes directions.

En particulier, l'un des buts de l'invention est de réaliser une prothèse totale de gros orteil permettant de restituer, grâce à sa géométrie adaptée, une flexion plantaire de 10 degrés et une flexion dorsale de 60 degrés, cette prothèse devant répondre de manière satisfaisante aux pathologies dites d'hallux rigidus stade II et stade III, voire l'hallux valgus arthrosique.

L'invention a pour objet une prothèse totale métatarsophalangienne à coupe selon la revendication 1.

Cette double courbure des deux surfaces conjuguées d'articulation de la prothèse permet à celle-ci de s'adapter avantageusement à l'anatomie du patient, en permettant de réaliser deux surfaces présentant une excellente congruence, laquelle assure également un recentrage automatique du corps du bouton phalangien sur l'implant métatarsien.

Ainsi la prothèse selon l'invention permet, outre les amplitudes de mouvement précitées, une réaxation de la phalange par rapport au métatarsien.

Suivant un mode de réalisation avantageux de l'invention, la zone de plus grand rayon de courbure s'étend sur environ les deux tiers des surfaces articulaires de l'implant métatarsien et du plateau intercalaire.

A titre d'exemple numérique, ces rayons de courbure peuvent être par exemple sensiblement de 5 à 15mm environ pour le premier rayon de courbure, et de 30 à 50mm environ pour le second rayon de courbure.

Suivant une autre particularité de l'invention, la tige d'ancrage osseux de l'implant métatarsien est inclinée d'environ 100 degrés sur une surface distale plane d'appui osseux du corps dudit implant auquel cette tige est fixée.

Cette surface plane distale vient s'appliquer sur une surface de coupe correspondante du métatarsien, l'inclinaison de la tige sur le corps de l'implant métatarsien étant telle que lorsque la prothèse est posée, la tige d'ancrage est coaxiale à l'axe du métatarsien. Une telle angulation présente l'avantage d'éviter toute interférence de l'extrémité plantaire du corps de l'implant métatarsien avec l'articulation métatarso-sésamoïdienne.

Par ailleurs comme déjà indiqué, la prothèse visée par l'invention est du type à coupe qui présente l'avantage de permettre un positionnement précis de ses composants, grâce à un ancillaire de coupe adapté, facilitant les coupes osseuses.

Les rayons de courbure de l'implant métatarsien restituent un valgus dynamique de 10 degrés après implantation de la prothèse, par glissement de l'implant phalangien par rapport à l'implant métatarsien.

D'autres particularités et avantages de l'invention apparaîtront au cours de la description qui va suivre faite en référence aux dessins annexés qui en illustrent une forme de réalisation à titre d'exemple non limitatif.

La figure 1 est une vue en élévation latérale à échelle réduite du squelette d'un pied humain droit, montrant en particulier l'articulation métatarso-phalangienne du gros orteil.

La figure 2 est une vue mi-coupe longitudinale mi-élévation, dans un plan vertical, à échelle agrandie d'une forme de réalisation de la prothèse totale métatarso-phalangienne selon l'invention, installée sur une articulation métatarso-phalangienne.

La figure 3 est une vue en élévation de l'implant métatarsien de la figure 2 dans la direction de la flèche F, depuis l'extrémité de sa tige d'ancrage.

La figure 4 est une vue de l'implant métatarsien suivant la direction de la flèche K de la figure 2.

La figure 5 est une vue en perspective à échelle agrandie, du plateau intercalaire entre les implants métatarsien et phalangien.

La figure 6 est une vue du plateau intercalaire en coupe dans un plan dorsal, c'est-à-dire un plan perpendiculaire à celui de la figure 2.

La figure 7 est une vue en coupe axiale, à échelle agrandie, d'un composant de l'implant phalangien.

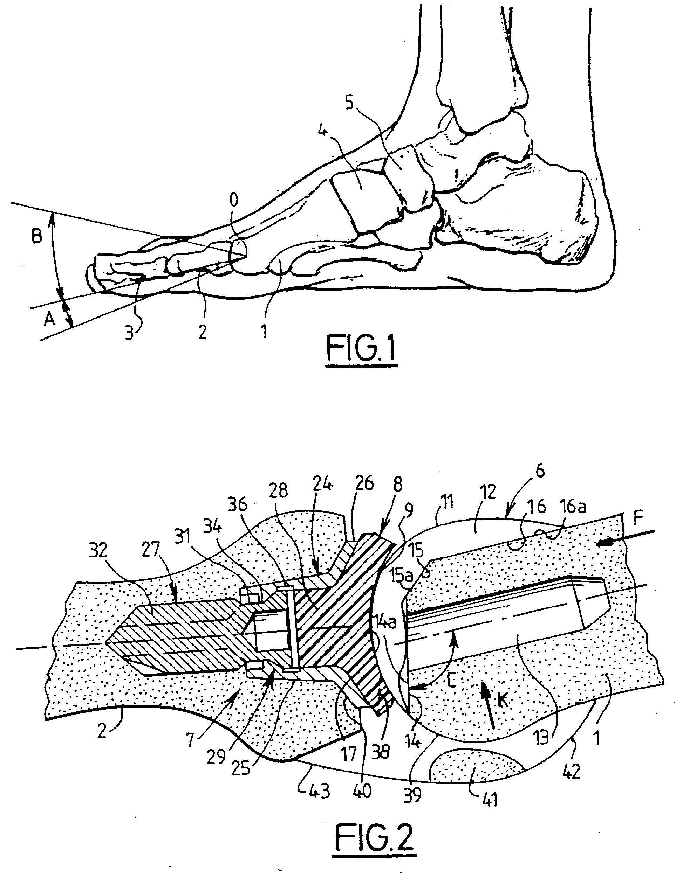

On voit à la figure 1 un squelette de pied humain droit, montrant le premier métatarsien 1, la phalange 2 articulée sur le métatarsien 1, la phalangette terminale 3 ainsi que les autres os du pied (premier cunéiforme 4, scaphoïde 5 etc.). La phalange 2 est articulée autour d'un point O, de part et d'autre duquel elle peut fléchir en entraînant la phalangette 3, soit en flexion plantaire (angle A), soit en flexion dorsale (B). La flexion plantaire maximum est de l'ordre de 10° et la flexion dorsale maximum B est de sensiblement 90° dans une articulation métatarso-phalangienne normale.

Le sésamoïde 41 est disposé (figure 2) sous la tête métatarsienne et relié au métatarsien 1 et à la phalange 2 par des tendons respectifs 42, 43.

La prothèse illustrée aux figures 2 à 7 est une prothèse totale à coupe, destinée à reproduire l'articulation entre le métatarsien 1 et la phalange 2. Cette prothèse totale comprend un implant métatarsien 6, un implant phalangien 7 et un plateau intermédiaire 8 disposé sur l'implant phalangien 7. Le plateau 8 présente une surface articulaire 9 coopérant avec une surface complémentaire d'articulation 11 ménagée sur un corps 12 de l'implant métatarsien 6. Ce dernier comprend également une tige 13 d'ancrage osseux fixée au corps 12, dont la surface articulaire 11 présente un contour arrondi, approximativement sphérique avec au moins trois rayons de courbure différents définis par une étude anatomique. Le corps 12 possède dans un plan vertical (figure 2) une section sensiblement en L.

Par contre la face interne du corps 12, située du côté de la tige d'ancrage 13, est segmentée en trois surfaces planes 14, 15, 16 usinées pour pouvoir venir s'appliquer avec précision sur des coupes distale 14a et dorsale 16a correspondantes du métatarsien 1. La surface intermédiaire 15 vient s'appliquer sur un chanfrein osseux 15a pratiqué entre les coupes dorsale et distale. La partie dorsale du corps 12 a une longueur approchant celle de la tige dorsale 13, laquelle forme avantageusement, avec le plan de la surface distale 14, un angle C d'environ 100°. L'axe de la tige 13 est ainsi coaxial à l'axe du métatarsien 1 lorsque la prothèse est posée.

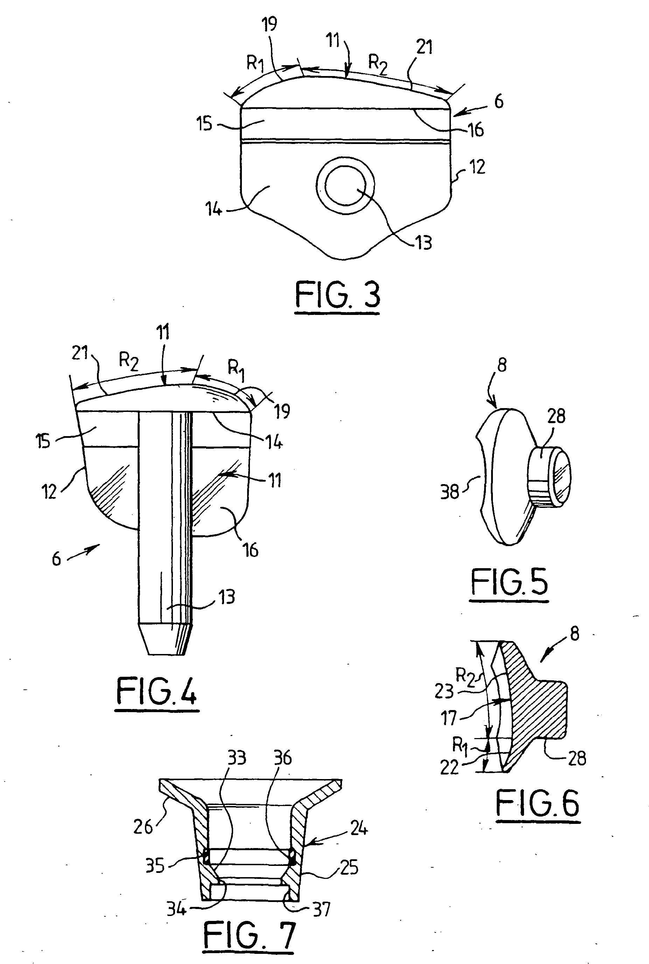

Dans un plan dorsal, c'est-à-dire dans le plan des figures 4 et 6, qui est perpendiculaire à celui de la figure 2, la surface articulaire 17 du plateau intercalaire 8 ainsi que la surface articulaire complémentaire 11 du corps 12 de l'implant métatarsien 6 présentent chacune deux zones de rayons de courbure différents, correspondant respectivement à l'anatomie du métatarsien 1 et de la phalange 2. Ainsi la surface articulaire 11 est divisée sur toute sa longueur dans le plan sagittal (Fig.2), en une première zone 19 ayant un rayon de courbure R1, et en une seconde zone 21 de rayon de courbure R2 supérieur à R1. De même, la surface articulaire 17 du plateau 8, dont le contour est sensiblement circulaire, comporte une première zone 22 dont le rayon de courbure est égal à R1, et une seconde zone 23 dont le rayon de courbure nettement supérieur, est égal à R2. Avantageusement, les zones 19 et 22 s'étendent sur sensiblement un tiers de la surface totale des surfaces respectives 11, 17, les deux tiers des surfaces restantes étant constitués par les zones 21 et 23.

Les rayons R1 et R2 ont des valeurs comprises entre celles indiquées précédemment, soit respectivement 5 à 15 mm et 30 à 50mm en fonction de la taille du patient auquel la prothèse totale est destinée.

Cette géométrie reproduit au plus près possible l'anatomie du patient, et assure donc une excellente congruence des surfaces articulaires 17 et 11.

L'implant phalangien 7 comprend une embase tubulaire de révolution 24 (figures 2 et 7) en forme d'entonnoir, constituée de deux parties coniques coaxiales 25 et 26. La partie conique 26 a un diamètre supérieur à celui de la partie conique 25 et sert d'appui au plateau intermédiaire 8, ainsi qu'à l'implant phalangien 7 sur la résection osseuse phalangienne. Le plateau 8 est réalisé en une matière plastique à faible coefficient de frottement tel que le polyéthylène. L'implant phalangien 7 comprend également un organe d'ancrage osseux constitué dans l'exemple représenté par une vis 27 à os spongieux, dont les filets sont à cet effet très profonds.

Le plateau intermédiaire 8 est complété par un plot axial 28 venu de matière avec le reste du plateau, et adapté pour s'engager axialement dans la partie conique 25 de l'embase phalangienne 24, afin de remplir une fonction anti-migration radiale du plateau 8 par rapport à l'implant phalangien 7. Cet agencement autorise par contre un débattement du plateau 8 axialement à l'embase 24. Par ailleurs, l'embase phalangienne 24 est intérieurement profilée pour pouvoir recevoir la vis à os spongieux 27 avec un jeu angulaire prédéterminé.

La vis 27 est constituée d'une tête fraisée 29 dont la surface conique se raccorde à une partie cylindrique 31 (figure 2) prolongée par la tige filetée 32. A ce profil conique et cylindrique de la tête fraisée 29 et de la zone 31, correspond un profil conjugué intérieur à la partie conique 25 de l'embase 24, constitué d'une paroi conique 33 (figure 7) se terminant par une collerette 34. Cette dernière se prolonge en direction de la vis 27 par un collet terminal 37 de diamètre supérieur à celui de la collerette 34.

La paroi conique 33 est raccordée, en direction de l'extrémité évasée 26, à une gorge 35 dans laquelle est logé un joint 36 assurant une rétention de la vis 27, ce joint 36 étant réalisé par exemple en silicone. Comme on le voit à la figure 2, la tête fraisée 29 vient prendre appui sur la partie conique 33 avec compression du joint 36. Cette compression assure une rétention de la vis 27 qui empêche son échappement après qu'elle ait été introduite à l'intérieur de l'embase 24 jusqu'à sa position illustrée à la figure 2.

En outre, la collerette intérieure 34 a un diamètre légèrement supérieur à celui de la partie cylindrique lisse 31, afin d'autoriser conjointement au diamètre du collet 37, un débattement angulaire limité de la vis 27 par rapport à l'embase phalangienne 24 après insertion de la vis dans celle-ci.

Lorsque la vis à os 27 est mise en place dans l'embase 24, sa tête 29 comprime le joint 36 qui assure ainsi une rétention de la vis 27.

Il est possible de ménager dans le bord du plateau 8 opposé au plan dorsal une échancrure 38 (figures 2 et 8) de dimension appropriée, dont l'avantage réside dans le fait qu'elle évite tout contact entre le bord du plateau 8 et la crête sésamoïdienne 39 de l'os métatarsien 1 lors d'une flexion plantaire, donc toute gêne ou douleur à cette occasion chez le patient.

Le plateau intermédiaire 8 peut être réalisé en plusieurs épaisseurs afin de constituer un jeu approprié dans lequel le chirurgien peut choisir le plateau le plus adapté au patient. Lé plot anti-migration 28 peut à titre d'exemple avoir une longueur d'environ 3mm. L'embase phalangienne 24 est de préférence recouverte extérieurement d'une couche d'hydroxyapatite, qui garantit une fixation stable sans utilisation de ciment chirurgical.

Outre les avantages mentionnés ci-dessus de la prothèse totale selon l'invention, celle-ci présente les suivants :
- la possibilité d'une rotation axiale relative entre le plateau intermédiaire 8 et l'embase phalangienne 24 permet de reproduire la rotation naturelle entre la phalange et le métatarsien dans la marche d'un individu. Cette liberté de rotation axiale autorise également l'auto-centrage automatique entre le plateau intermédiaire 8 et l'implant métatarsien 6, lui même lié à la double courbure R1, R2 des surfaces articulaires.
- La rétention de la vis à os 27 dans l'embase 24 assurée par le joint 36 facilite la pose par le chirurgien en permettant à celui-ci de manipuler un implant phalangien 7 quasi monobloc. Elle évite de plus que la vis 27 ne recule pour venir au contact du plot 28 en polyéthylène après la pose, provoquant une usure indésirable du plot 28. Cette rétention par le joint 36 autorise néanmoins une faible angulation, de l'ordre de ± 3° permettant une orientation automatique de la vis 27 dans la phalange 2.
- L'échancrure 38 dans la partie plantaire du plateau 8 évite toute interférence avec la crête sésamoïdienne et indexe le placement du plateau 8 par rapport à l'implant métatarsien 6.
- Le plot 28 évite tout risque de migration du plateau 8 par rapport à l'embase phalangienne 28 dans laquelle il prend place.
- L'implant phalangien 7 est solidement ancré par la vis 27 dans la phalange 2, et son plateau intermédiaire 8 restitue la surface articulaire de la base de la phalange.
- Le mouvement de rotation axiale entre le plateau 8 et l'implant phalangien 7 génère un volume de débris très faible et en pratique négligeable, dans des conditions physiologiques.

La surface articulaire 11 de l'implant métatarsien 6 restitue la bande de glissement initialement réalisée par la tête métatarsienne. Dans un plan de profil, la coupe distale métatarsienne doit être angulée de 10° environ par rapport à un plan perpendiculaire à l'axe diaphysaire. Cette coupe plonge vers la face plantaire du métatarsien 1 et vient effleurer l'emplacement du sésamoïde 41 sur le métatarsien, afin d'être parallèle à la coupe phalangienne. La coupe dorsale du métatarsien 1 vient araser la corticale dorsale du métatarsien, de sorte que l'implant 6 comporte trois plans de coupe : la coupe dorsale du plan 16 , la coupe distale 14, et une coupe de chanfrein correspondant à la surface 15, par exemple de 2mm entre les deux coupes précédentes. L'implant 6 comporte des rayons de courbure permettant de couvrir l'ensemble des morphotypes, sa longueur en vue dorsale devant également répondre à ce critère.

L'invention est susceptible de diverses variantes d'exécution. Ainsi la proportion un tiers/deux tiers entre les deux zones de rayons de courbure R1 et R2 peut sensiblement varier en fonction de l'anatomie du patient, de même que les valeurs numériques des rayons de courbure. La rétention de la vis à os 27 dans l'embase 24 peut être réalisée par tout moyen équivalent au joint rétentif 36.

## Revendications

1. Prothèse totale métatarso-phalangienne à coupe, comprenant un implant métatarsien (6) constitué d'un corps (12) et d'une tige d'ancrage osseux (13), un implant phalangien (7) et un plateau intermédiaire (8) adapté pour être disposé sur l'implant phalangien et pourvu d'une surface articulaire (17) coopérant avec une surface complémentaire (18) d'articulation du corps de l'implant métatarsien, **caractérisée en ce que** lesdits surfaces articulaire et complémentaire d'articulation sont divisées chacune en deux zones (22 , 23 ; 19, 21) sur toute leur longueur dans le plan sagittal et ayant respectivement des rayons de courbure différents (R1, R2) correspondant respectivement à l'anatomie du métatarsien (1) et de la phalange (2), et
**en ce que** la zone de plus grand rayon de courbure (R2) s'étend sur environ les deux tiers des surfaces articulaires (18, 17) de l'implant métatarsien (6) et du plateau (8), ledit rayon de courbure (R2) étant compris entre 30 et 50 mm, alors que le rayon de courbure plus faible (R1) est compris entre 5 et 15 mm.

2. Prothèse selon la revendication 1, **caractérisée en ce que** l'implant phalangien (6) comprend une embase tubulaire (24) de révolution en forme d'entonnoir montée sur un organe (27) d'ancrage osseux, et sur laquelle prend appui le plateau (8) qui peut tourner par rapport à l'embase phalangienne.

3. Prothèse selon la revendication 2, **caractérisée en ce que** l'organe d'ancrage osseux est une vis (27) à os spongieux.

4. Prothèse selon la revendication 3, **caractérisée en ce que** le plateau (8) est muni d'un plot (28) antimigration adapté pour s'engager axialement dans l'embase phalangienne (24).

5. Prothèse selon les revendications 2 et 3, **caractérisée en ce que** l'embase (24) est intérieurement profilée afin de pouvoir recevoir la vis (27) à os spongieux avec un jeu angulaire.

6. Prothèse selon la revendication 5, **caractérisée en ce que** l'embase (24) présente une collerette intérieure (34) dont le diamètre est légèrement supérieur à celui d'une partie cylindrique lisse (31) de la vis (27) à os spongieux, située entre la tête (29) et la partie filetée (32) de ladite vis.

7. Prothèse selon la revendication 2, **caractérisée en ce que** des moyens de rétention sont prévus pour retenir l'organe d'ancrage osseux (27) sur l'embase phalangienne (24) afin de l'empêcher de venir en contact avec le plateau (8).

8. Prothèse selon les revendications 2, 3 et 7, dans laquelle la tête (29) de la vis à os (27) est fraisée, **caractérisée en ce que** lesdits moyens de rétention comprennent un joint souple (36) logé dans une gorge intérieure (35).

## Claims

1. Total metatarsophalangeal prosthesis with section plane, comprising a metatarsal implant (6) formed by a body (12) and an anchoring bar (13) of bone, a phalangeal implant (7) and an intermediate plate (8) adapted to be placed on the phalangeal implant and provided with an articular surface (17) cooperating with a complementary surface (18) for articulation of the body of the metatarsal implant, **characterised in that** said articular and complementary articulation surfaces are each divided into two zones (22, 23; 19, 21) over their entire length in the sagittal plane and each have different radii of curvature (R1, R2) to correspond to the anatomy of the metatarsal (1) and the phalange (2) respectively, and
**in that** the zone with the larger radius of curvature (R2) extends over approximately two thirds of the articular surfaces (18, 17) of the metatarsal implant (6) and of the plate (8), said radius of curvature (R2) being in the range of between 30 and 50 mm, whereas the smaller radius of curvature (R1) is in the range of between 5 and 15 mm.

2. Prosthesis according to Claim 1, **characterised in that** the phalangeal implant (6) comprises a tubular revolving seat (24) in the shape of a funnel mounted on an anchoring element (27) of bone, and on which the plate (8) rests, which can rotate in relation to the phalangeal seat.

3. Prosthesis according to Claim 2, **characterised in that** the anchoring element of bone is a spongy bone screw (27).

4. Prosthesis according to Claim 3, **characterised in that** the plate (8) is fitted with a migration inhibiting stud (28) adapted to engage axially into the phalangeal scat (24).

5. Prosthesis according to Claims 2 and 3, **characterised in that** the seat (24) is profiled on the inside to enable it to receive the spongy bone screw (27) with angular play.

6. Prosthesis according to Claim 5, **characterised in that** the seat (24) has an internal collar (34), the diameter of which is slightly larger than that of a smooth cylindrical section (31) of the spongy bone screw (27) located between the head (29) and the threaded section (32) of said screw.

7. Prosthesis according to Claim 2, **characterised in that** holding means are provided to hold the anchoring element (27) of bone on the phalangeal seat (24) to prevent it coming into contact with the plate (8).

8. Prosthesis according to Claims 2, 3 and 7, in which the head (29) of the bone screw (27) is countersunk, **characterised in that** said holding means comprise a flexible sealing strip (36) housed in an internal throat (35).

## Patentansprüche

1. Metatarsalphalangeale Totalprothese für vorgeschnittene Knochenoberflächen, umfassend ein Metatarsalimplantat (6), das aus einem Körper (12) und aus einer Knochenverankerungsstange (13) besteht, ein Phalangealimplantat (7) und eine Zwischenplatte (8), die dafür ausgelegt ist, auf dem Phalangealimplantat angeordnet zu werden, und mit einer Gelenkfläche (17) versehen ist, die mit einer ergänzenden Fläche (18) zur Anlenkung des Körpers des Metatarsalimplantats zusammenwirkt, **dadurch gekennzeichnet, dass** die Gelenkfläche und die ergänzende Anlenkungsfläche jeweils auf ihrer ganzen Länge in der Sagittalebene in zwei Zonen (22, 23; 19, 21) unterteilt sind und jeweils verschiedene Krümmungsradien (R1, R2) besitzen, die der Anatomie des Metatarsus (1) bzw. der Phalanx (2) entsprechen, und
dass die Zone mit größerem Krümmungsradius (R2) sich über etwa zwei Drittel der Gelenkflächen (18, 17) des Metatarsalimplantats (6) und der Platte (8) erstreckt, wobei dieser Krümmungsradius (R2) zwischen 30 und 50 mm beträgt, während der kleinere Krümmungsradius (R1) zwischen 5 und 15 mm beträgt.

2. Prothese nach Anspruch 1, **dadurch gekennzeichnet, dass** das Phalangealimplantat (6) einen rotationskörperförmig rohrförmigen Sockel (24) in Trichterform umfasst, der auf einem Knochenverankerungsorgan (27) montiert ist und auf dem die Platte (8) aufliegt, die sich bezüglich des Phalangealsockels drehen kann.

3. Prothese nach Anspruch 2, **dadurch gekennzeichnet, dass** das Knochenverankerungsorgan eine Spongiosaschraube (27) ist.

4. Prothese nach Anspruch 3, **dadurch gekennzeichnet, dass** die Platte (8) mit einem Antimigrationszapfen (28) versehen ist, der dafür ausgelegt ist, in den Phalangealsockel (24) axial eingesteckt zu werden.

5. Prothese nach Anspruch 2 und 3, **dadurch gekennzeichnet, dass** der Sockel (24) innen profiliert ist, um die Spongiosaschraube (27) mit einem Winkelspiel aufnehmen zu können.

6. Prothese nach Anspruch 5, **dadurch gekennzeichnet, dass** der Sockel (24) eine Innenschulter (34) aufweist, deren Durchmesser etwas größer als der eines glatten zylindrischen Teils (31) der Spongiosaschraube (27) ist, der zwischen dem Kopf (29) und dem Gewindeteil (32) dieser Schraube gelegen ist.

7. Prothese nach Anspruch 2, **dadurch gekennzeichnet, dass** Rückhaltemittel vorgesehen sind, um das Knochenverankerungsorgan (27) auf dem Phalangealsockel (24) zurückzuhalten, um es daran zu hindern, mit der Platte (8) in Kontakt zu kommen.

8. Prothese nach den Ansprüche 2, 3 und 7, bei der der Kopf (29) der Knochenschraube (27) gefräst ist, **dadurch gekennzeichnet, dass** die Rückhaltemittel einen biegsamen Ring (36) umfassen, der in einer inneren Nut (35) sitzt.
